(19) Europäisches Patentamt
European Patent Office
Office européen des brevets

(11) **EP 2 949 957 A1**

(12) **EUROPEAN PATENT APPLICATION**
published in accordance with Art. 153(4) EPC

(43) Date of publication:
02.12.2015 Bulletin 2015/49

(51) Int Cl.:
*F16D 25/12* (2006.01)    *F16H 59/72* (2006.01)

(21) Application number: 13872339.0

(22) Date of filing: 28.11.2013

(86) International application number:
PCT/JP2013/082000

(87) International publication number:
WO 2014/115424 (31.07.2014 Gazette 2014/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 24.01.2013 JP 2013011051

(71) Applicant: Jatco Ltd
Fuji-shi, Shizuoka 417-8585 (JP)
(72) Inventors:
• KAWAKAMI, Hiroki
Fuji-shi
Shizuoka 417-8585 (JP)
• MURAMOTO, Atsumi
Fuji-shi
Shizuoka 417-8585 (JP)
• MIURA, Kota
Fuji-shi
Shizuoka 417-8585 (JP)

(74) Representative: Grünecker Patent- und
Rechtsanwälte
PartG mbB
Leopoldstraße 4
80802 München (DE)

(54) **TEMPERATURE ESTIMATION CALCULATION DEVICE FOR FRICTIONAL ENGAGEMENT ELEMENT**

(57)    For clutch (23) in vehicle driving force transmitting system, having operating states of full engagement, slip engagement and disengagement, there are provided heat generation calculating section (32) calculating per-unit-time temperature increase ΔT(up) from heat generation quantity (Σt) generated among friction material members of clutch (23), heat dissipation calculating section (33) calculating per-unit-time temperature decrease ΔT(down) from heat dissipation quantity f(t) dissipated from the friction material of clutch (23), and clutch temperature estimation calculating process section (31) calculating current clutch temperature Tnow by adding temperature variation ΔT to previous clutch temperature Tpv. Heat dissipation calculating section (33) estimates a quantity of lubricating oil passing around the friction material of clutch (23) and uses the estimated lubricating oil quantity for the heat dissipation quantity f(t) dissipated by heat transfer from the friction material to the lubricating oil.

**FIG.5**

EP 2 949 957 A1

**Description**

**TECHNICAL FIELD**

The present invention relates to apparatus or device for estimation and calculation of a temperature of a friction engagement element disposed in a driving force transmitting system of a vehicle and formed by a friction clutch or a friction brake having operating states of full engagement/slip engagement/disengagement.

**BACKGROUND ART**

A known apparatus for calculating a surface temperature of a vehicle friction engagement device is arranged to correct an accumulated cooling temperature in accordance with a per-unit-time heat generation quantity of a lockup clutch and its turbine rotational speed, and thereby to improve a calculating accuracy of the surface temperature (cf. Patent Document 1, for example).

**PRIOR ART DOCUMENT(S)**

**Patent Document(s)**

Patent Document 1: JP2011-99513A

**SUMMARY OF THE INVENTION**

**Problem to be solved by the invention**

Though the known vehicular friction engagement device surface temperature calculating apparatus is arranged to estimate the temperature in consideration of the effect of heat dissipation by the turbine rotational speed, the known surface temperature calculating apparatus does not take account of the effect of heat dissipation due to a quantity of lubricating oil passing around the friction material of the lockup clutch. Accordingly, the calculated value of the clutch surface temperature may be deviated largely from an actually measured value.

The present invention has been devised in view of this problem, and an object of the present invention is to provide an apparatus for estimating and calculating temperature of a friction engagement element, for improving the accuracy of calculation of estimating the temperature of the friction engagement element by taking account of the effect of heat dissipation by the lubricating oil passing round the friction material.

**Means for solving the Problem**

In order to achieve the above-mentioned object, according to the present invention, as to a friction engagement element disposed in a vehicular driving force transmitting system, and arranged to have operating states of full engagement, slip engagement and disengagement, there are provided a heat generation calculating means, a heat dissipation calculating means and a friction engagement estimated temperature calculating means.

The heat generation calculating means calculates a temperature increase per unit time in accordance with a heat generation quantity of heat generated among friction material members of the friction engagement element.

The heat dissipation calculating means calculates a temperature decrease per unit time in accordance with a heat dissipation quantity of heat dissipated from the friction material of the friction engagement element.

The friction engagement element estimated temperature calculating means sets a temperature difference resulting from subtraction of the temperature decrease from the temperature increase, as a temperature variation per unit time, and calculates a current friction engagement element estimated temperature by adding the temperature variation to a previous friction engagement estimated temperature estimated until a previous calculating cycle.

The heat dissipation calculating means estimates a lubricating oil quantity of a lubricating oil passing around the friction material of the friction engagement element, and calculates the heat dissipation quantity in accordance with the estimated lubricating oil quantity.

**Effect(s) of the Invention**

Thus, the friction engagement element estimated temperature calculating means sets the temperature difference resulting from subtraction of the temperature decrease caused by the heat dissipation from the temperature increase caused by the heat generation, as the temperature variation per unit time, and calculates the current friction engagement element estimated temperature by adding the temperature variation to the previous friction engagement estimated temperature estimated until a previous calculating cycle. In this case, in the heat dissipation calculating means, the lubricating oil quantity of the lubricating oil passing around the friction material of the friction engagement element is estimated, the heat dissipation quantity is calculated in accordance with the lubricating oil quantity obtained by estimation, and the per-unit-time temperature decrease is calculated in accordance with the heat dissipation quantity.

Frictional heat generated in the friction material is dissipated or released to the lubricating oil by the action of heat transfer from the friction material to the lubricating oil during the passage of the lubricating oil around the friction material. Therefore, if the temperature decrease caused by this heat dissipation is not taken into consideration, the estimated temperature of the friction engagement element would be too high.

By the incorporation of the quantity of the lubricating oil flowing around the friction material into the calculation of heat dissipation, by contrast, the quantity of heat released by the heat transfer from the friction material to the lubricating oil is reflected in the temperature estimation of the friction engagement element made up of the heat generation calculation and the heat dissipation calculation.

Thus, by taking account of the heat dissipating effect by the lubricating oil passing around the friction material, it is possible to improve the accuracy of estimation for estimating the temperature of the friction engagement element.

## BRIEF DESCRIPTION OF THE DRAWINGS

FIG. 1 is an overall system view showing a drive system and a control system of a rear wheel drive vehicle (as an example of a vehicle) to which a friction engagement element temperature estimation calculating apparatus according to an embodiment 1.

FIG. 2 is a flowchart showing a flow of a clutch temperature estimation calculating process performed in a clutch temperature estimation calculating process section of an AT controller according to the embodiment 1.

FIG. 3 is a block diagram showing a heat generation calculating section in the clutch temperature estimation calculating section of the AT controller according to the embodiment 1.

FIG. 4 is a characteristic view of oil pressure and heat generation quantity characteristics showing a situation or variation of heat accumulation and heat generation during a shift, calculated by the heat generation calculating section.

FIG. 5 is a block diagram a showing a heat dissipation calculating section in the clutch temperature estimation calculating section of the AT controller according to the embodiment 1.

FIG. 6 is a view showing one example (a) of a line pressure sensitivity map for determining a line pressure sensitivity of a lubrication quantity with respect to a line pressure in a lubrication calculation in a lubrication quantity calculating section, and one example (b) of a surface pressure sensing map for determining a surface pressure sensitivity of the lubrication quantity with respect to a solenoid regulation pressure.

FIG. 7 is a view showing one example of a heat dissipation quantity map for determining a map selection coefficient used in the calculation of the heat dissipation quantity in a heat dissipation quantity calculating section.

FIG. 8 is a view for illustrating the relation of lubricating oil quantity estimating factor(s) with respect to the lubrication quantity around the friction material used as a heat dissipation calculating base according to the embodiment 1.

FIG. 9 is a view illustrating the flow of the lubricating oil around he friction material in the disengagement state (a), the slip engagement state (b) and the full engagement state (c).

FIG. 10 is a view showing a comparative characteristic view (a) showing an increase of the clutch temperature with time in the heat generation calculation in the embodiment 1, and a schematic view (b) illustrating heat dissipated or released from the friction material during the heat generation, through a partner member, to the outside.

## MODE(S) FOR CARRYING OUT THE INVENTION

Hereinafter, best mode(s) implementing a friction engagement element temperature estimation calculating apparatus according to the present invention is explained by using an embodiment 1 illustrated in the drawings.

## Embodiment 1

Construction is explained first. The friction engagement element temperature estimation calculating apparatus of the embodiment 1 is explained by using divisions of "Overall System Construction", "Clutch Temperature Estimation Calculation Process Construction", "Heat Generation Calculation Construction" and "Heat Dissipation Calculation Construction".

[Overall System Construction]

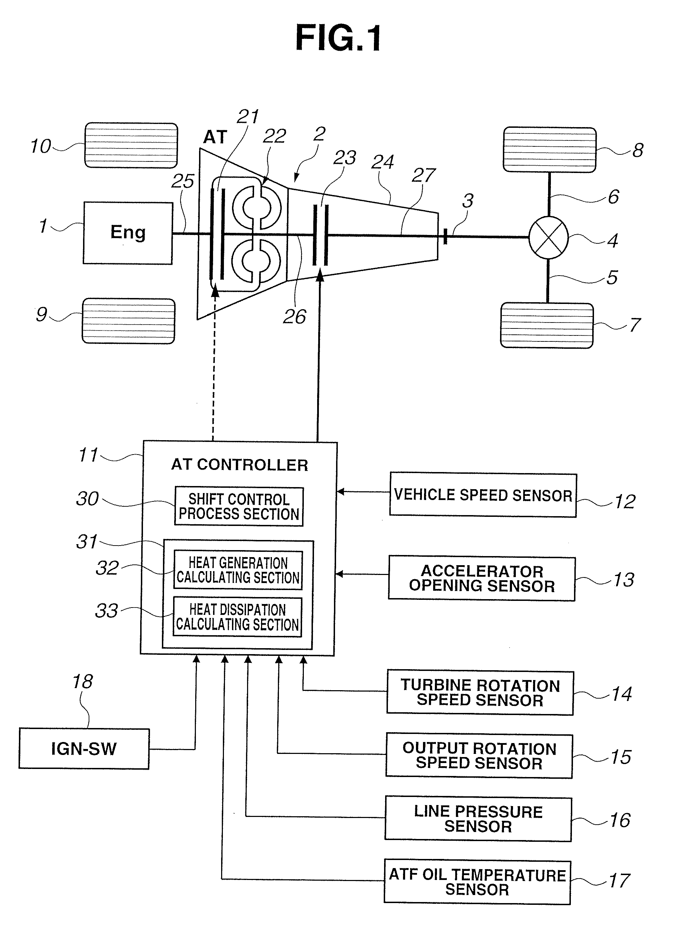

FIG. 1 shows a drive system and a control system of a rear wheel drive vehicle (as an example of a vehicle) to which the friction engagement element temperature estimation calculating apparatus of the embodiment 1 is applied. Hereinbelow, the overall system construction is explained by using FIG. 1.

As shown in FIG. 1, the drive system of the rear wheel drive vehicle includes an engine 1, an automatic transmission 2, a propeller shaft 3, a differential 4, a left drive shaft 5, a right drive shaft 6, a left rear wheel 7 and a right rear wheel 8. A left front wheel 9 and a right front wheel 10 are non-driven follower wheels.

Automatic transmission 2 includes a torque converter 22 having a lockup clutch 21, a plurality of clutches 23 as shifting elements, and a gear train. Automatic transmission 2 is provided with a shift mechanism 24 for changing the shit state from one to another among a plurality of stepwise speeds or engagement positions, automatically by a clutch engagement changeover control. The lockup clutch 21 is interposed between an engine output shaft 25 and a transmission input shaft 26. Each clutch 23 is interposed in a torque transmission path at the selected speed, between the transmission input shaft 26 and a transmission output shaft 27. FIG. 1 shows only one of clutches 23.

The object of a drive system temperature estimation calculation may be any of the lockup clutch 21 and the clutches 23 as a friction engagement element interposed in a driving force transmission path and arranged to attain full engagement, slipping or slip engagement and disengagement or release. In the following explanation of the embodiment 1, the object of the drive system temperature estimation calculation is the clutch(s) 23 disposed in the shift mechanism 24 and formed by a wet type multiple plate friction clutch or a multiple plate friction brake.

As shown in FIG. 1, the control system of the rear wheel drive vehicle includes an AT controller 11, a vehicle speed sensor 12, an accelerator opening sensor 13, a turbine rotation speed sensor 14, an output rotation speed sensor 15 sensing an output side rotation speed No of the clutch 23, a line pressure sensor 16, an ATF oil temperature sensor 17 and an ignition switch 18.

The AT controller 11 includes a shift control process section 30 for controlling upshift and downshift, and a clutch temperature estimation calculating process section 31 including a heat generation calculating section 32 and a heat dissipation calculating section 33.

The vehicle speed sensor 12 and accelerator opening section 13 are used for providing shifting information determining a current operation point on an unillustrated shift map having upshift line(s) and downshift line(s). The shift map is a map using the vehicle speed and accelerator opening as coordinate axes, and including preliminary set upshift line(s) and downshift line(s).

The turbine rotation speed sensor 14, output rotation speed sensor 15, line pressure sensor 16 and ATF oil temperature sensor 17 are used for providing information on clutch relative rotation speed, information on line pressure and information on ATF oil temperature for the clutch temperature estimation calculation. The line pressure PL is an original oil pressure for the clutch(s) 23. The line pressure is produced by pressure regulation by an unillustrated pressure regulator valve in accordance with conditions such as the accelerator opening. A drain oil is produced simultaneously with the line pressure at the time of pressure regulating operation by the pressure regulator valve. The flow quantity of the drain oil is increased as the line pressure PL becomes higher. The drain oil is used as the lubricating oil for clutch(s) 23. The ATF temperature is the temperature of a transmission operating oil (ATF) used as the shifting oil pressure and the lubrication in the shift mechanism 24.

[Clutch Temperature Estimation Calculation Process Construction]

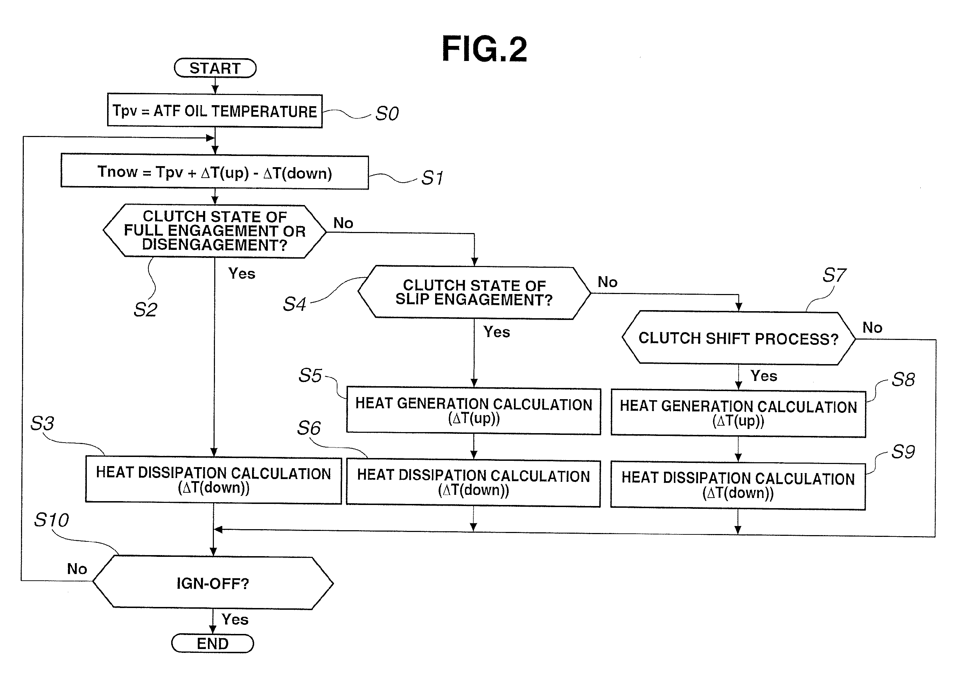

FIG. 2 is a flowchart showing a flow of a clutch temperature estimation calculating process performed in the clutch temperature estimation calculating process section 31 of AT controller 11. Following is explanation on steps representing the clutch temperature estimation calculating process construction. The calculation process is repeated every predetermined calculation cycle.

At a step S0, the controller sets an initial clutch temperature or clutch temperature initial value equal to the ATF oil temperature supplied from ATF oil temperature sensor 17 at a start by an ignition switch on operation. Thereafter, the controller proceeds to a step S1.

At the step S1 following the setting of Tpv=ATF oil temperature at step S0 or the judgment at a step S10 that the ignition switch 18 is ON, the controller estimates and calculates a current clutch temperature or current value of the clutch temperature Tnow by adding, to the previous clutch temperature Tpv, a temperature variation $\Delta T$ obtained by subtraction, from a temperature increase $\Delta T(up)$ obtained by a heat generation calculation, of a temperature decrease $\Delta T(down)$ obtained by a heat dissipation calculation (friction engagement element estimation temperature calculating means). Thereafter, the controller proceeds to a step S2.

When only the heat dissipation calculation is performed, the current clutch temperature Tnow is estimated and calculated by subtracting the temperature decrease $\Delta T(down)$ from the previous clutch temperature Tpv (by setting $\Delta T(up)$ equal to zero in the mathematical expression at S1). If the calculated current clutch temperature Tnow is lower than or equal to the ATF oil temperature, the controller initializes the clutch temperature (the current clutch temperature Tnow=the ATF oil temperature), and manages the clutch temperature estimation calculation by using the ATF oil temperature as a lower limit temperature.

At the step S2 following the estimation calculation of current clutch temperature Tnow at step S1, the controller examines whether clutch 23 is in a state of full engagement or disengagement, or not. The controller proceeds to a step S3 in the case of YES (the full engagement /disengagement state), and proceeds to a step S4 in the case of NO (the state other than the full engagement/disengagement state).

In this case, the clutch is judged to be in the full engagement state when the command from AT controller 11 to clutch 23 is an engagement command with the line pressure PL. The clutch is judged to be in the disengagement state when the command of AT controller 11 to clutch 23 is a clutch disengagement command.

At the step S3 following the judgment of the full engagement/disengagement sate at step S2, the controller calculates a per-unit-time temperature decrease $\Delta T$(down) in accordance with a heat dissipation quantity of heat dissipated or released from the friction material of clutch 23 (heat dissipation calculating means). Thereafter, the controller proceeds to the step S10.

In this heat dissipation calculation, the controller estimates a quantity of lubricating oil passing around the friction material of clutch 23 in the full engagement state or in the disengagement state. In accordance with the thus-estimated lubricating oil quantity, the controller calculates the heat dissipation quantity of heat dissipated or released by heat transfer from the friction material to the lubricating oil. The heat dissipation calculation at steps S3, S6 and S9 is explained in

detail later.

At the step S4 following the judgement of the state other than the full engagement/disengagement state at S2, the controller examines whether clutch 23 is in the slip engagement state or not. The controller proceeds to a step S5 in the case of Yes (in the slip engagement state), and to a step S7 in the case of No (not in the slip engagement state).

At the step S5 following the judgment at step S4 that clutch 23 is in the slip engagement state, the controller calculates a per-unit-time temperature increase $\Delta T$(up) in accordance with a heat generation quantity of heat generated between members of the friction material of clutch 23 (heat generation calculating means). Thereafter, the controller proceeds to the step S6.

In this heat generation calculation, the controller takes account of a quantity of heat released from a heat generating portion between both plates during slipping heat generation, to the outside through base plate(s). The heat generation calculation at steps S5 and S8 is explained in detail later.

At the step S6 following the heat generation calculation at step S5, the controller calculates the per-unit-time temperature decrease $\Delta T$(down) in accordance with the heat dissipation quantity of heat dissipated or released from the friction material of clutch 23 (the heat dissipation calculating means). Thereafter, the controller proceeds to the step S10. In this heat dissipation calculation, the controller estimates the quantity of lubricating oil passing around the friction material of clutch 23 in the slip engagement state, and calculates the heat dissipation quantity of heat dissipated or released by heat transfer from the friction material to the lubricating oil, by using the thus-estimated lubricating oil quantity.

At the step S7 following the judgement at step S4 that the clutch is in the state other than the slip engagement state, the controller examines whether clutch 23 is in a state of shift process. The controller proceeds to the step S8 in the case of Yes (during the shift process), and to the step S10 in the case of No (not in the state of the shift process). The controller follows a predetermined fail-safe process when the judgement at step S7 is that the clutch is in the state other than the state of the shift process.

At the step S8 following the judgment at step S7 that clutch 23 is in the state of the shift process, the controller calculates the per-unit-time temperature increase $\Delta T$(up) in accordance with the heat generation quantity of heat generated between the members of the friction material of clutch 23 (heat generation calculating means). Thereafter, the controller proceeds to the step S9. In this heat generation calculation, the controller takes account of the quantity of heat released from the heat generating portion between both plates during the shift operation, to the outside through the base plate(s).

At the step S9 following the heat generation calculation at step S8, the controller calculates the per-unit-time temperature decrease $\Delta T$(down) in accordance with the heat dissipation quantity of heat dissipated from the friction material of clutch 23 (the heat dissipation calculating means). Thereafter, the controller proceeds to the step S10.

In this heat dissipation calculation, the controller estimates the quantity of lubricating oil passing around the friction material of clutch 23 in the state of the shift process, and calculates the heat dissipation quantity of heat dissipated by heat transfer from the friction material to the lubricating oil, by using the thus-estimated lubricating oil quantity.

At the step S10 following the heat dissipation calculation at step S3, S6 or S9, or the judgement at step S7 that the clutch is in the state other than the state of the shift process, the controller determines whether the ignition switch 18 is Off or not. The controller proceeds to END in the case of YES (IGN-OFF), and returns to step S1 in the case of NO (IGN-ON).

[Heat Generation Calculation Construction]

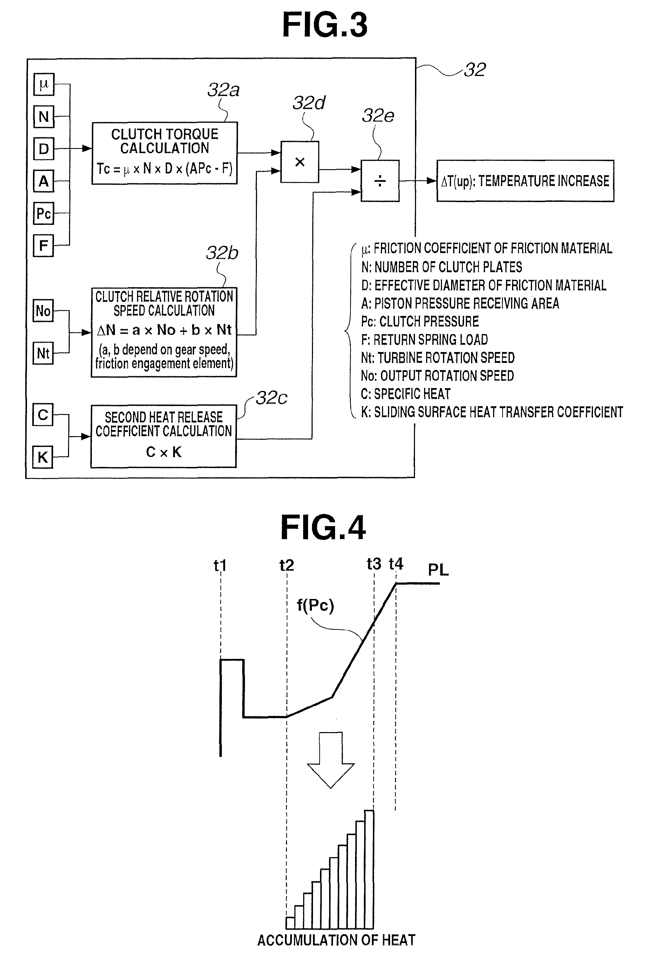

FIG. 3 shows the heat generation calculating section 32 of the clutch temperature estimation calculating section 31. FIG. 4 shows the heat generation quantity calculated by the heat generation calculating section 32 (a situation of heat accumulation and heat generation during shift). Following is explanation on the heat generation calculation construction based on FIGS 3 and 4.

As shown in FIG. 3, the heat generation calculating section 32 includes a clutch torque calculating section 32a, a clutch relative rotation calculating section 2b, a second heat release coefficient calculating section 32c, a heat generation quantity calculating section 32d and a dividing section 32e.

The clutch torque calculating section 32a calculates a clutch torque Tc according to a following equation,

$$Tc = \mu \times N \times D \times (A \times Pc - F)$$

where $\mu$: a friction coefficient of the friction material, N: a number of clutch plates, D: an effective diameter of the friction material, A: a piston pressure receiving area, Pc: a clutch pressure, and F: a return spring load. The clutch pressure Pc is estimated from the clutch pressure command outputted from AT controller 11.

The clutch relative rotation calculating section 32b calculates a clutch relative rotation speed or rpm $\Delta N$ according to a following equation,

$$\Delta N = a \times No + b \times Nt$$

where No: an output rotational speed or rpm, Nt: a turbine rotational speed or rpm, and a and b are coefficients varying in dependence on the gear speed and the friction engagement element.

The second heat release coefficient calculating section 32c calculates a second heat release coefficient according to a following mathematical expression,

$$C \times K$$

where C: a specific heat, and K: a sliding surface heat transfer coefficient.

The specific heat C is determined by the material used as the friction material. The sliding surface heat transfer coefficient K is determined as a fixed value (>1) based on a measured value obtained by measurement, by experiment or the like, of the quantity of heat released to the outside from both friction material members by heat transfer through the base plate(s) during heat generation due to slipping friction.

The heat generation quantity calculating section 32d calculates the heat generation quantity $\Sigma t$ according to a following equation,

$$\Sigma t = Tc \times \Delta N$$

where Tc: the clutch torque, and $\Delta N$: the clutch relative rotational speed.

As shown in FIG. 4, the heat generation quantity $\Sigma t$ of the friction engagement element engaged during the shift operation is a quantity of heat accumulated during a period from a clutch engagement start instant t2 determined by the clutch pressure command f(Pc) to a clutch engagement end instant t3 in a period from a shift start instant t1 to a shift end instant t4. Thus, the heat generation quantity $\Sigma t$ is determined by an integration process of multiplication of clutch torque Tc by clutch relative rotational speed $\Delta N$, and addition from moment to moment. Because of the integration, the quantity increases from a start of the relative rotation until an end at which the relative rotation is reduced to zero.

The dividing section 32e calculates the temperature increase $\Delta T(up)$ caused by the heat generation, according to a following equation of dividing the heat generation quantity $\Sigma t$ by the second heat release coefficient,

$$\Delta T(up) = \{\Delta N \times \mu ND \times (A \times Pc - F) \times \Delta t / 1000\} / C \times K \quad \cdots (1)$$

where $\Delta t$ is a calculating cycle, and the temperature increase $\Delta T(up)$ represents an increase of the temperature per unit time determined by the calculating cycle or period $\Delta t$.

[Heat Dissipation Calculation Construction]

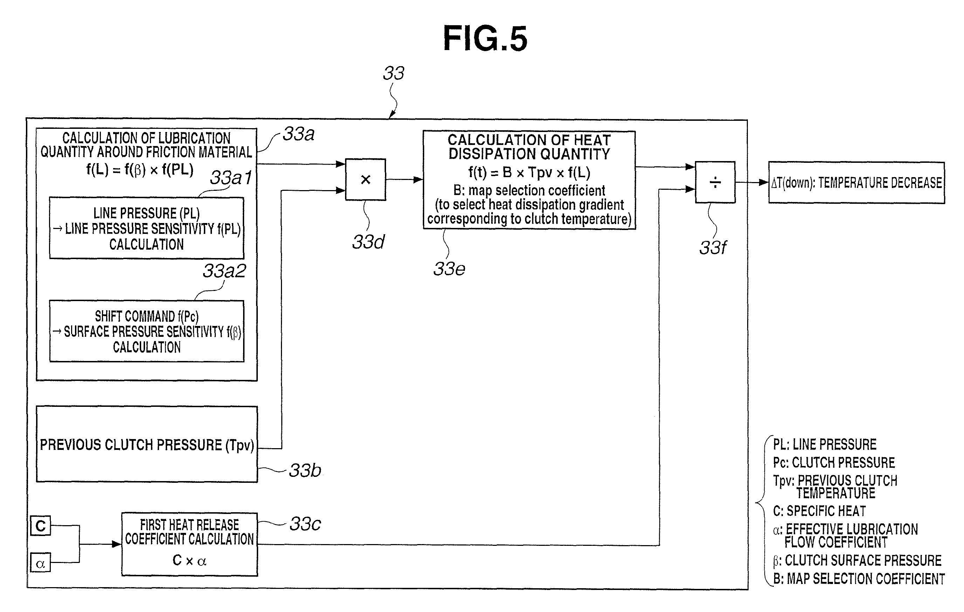

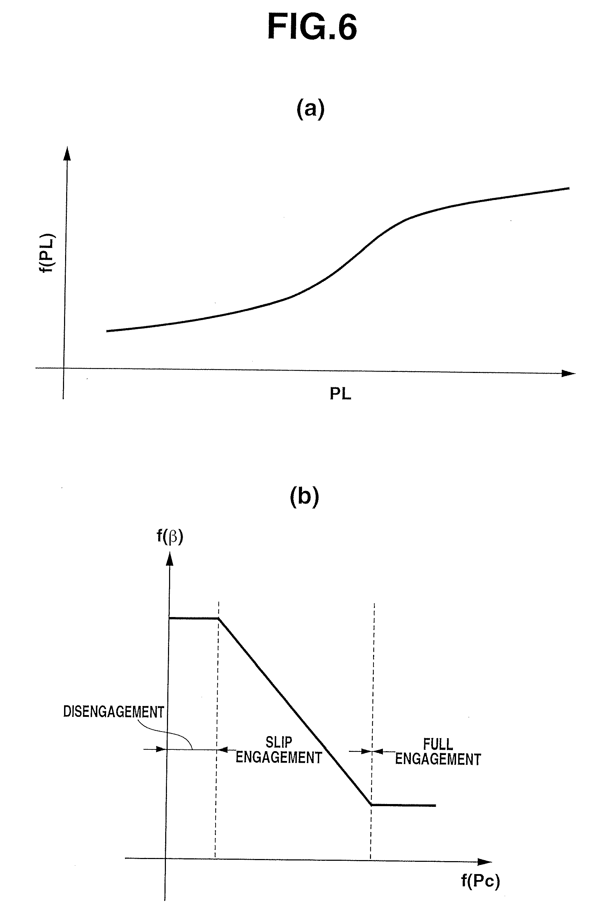

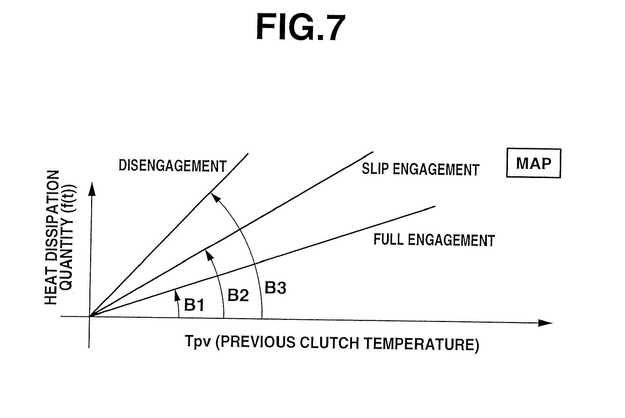

FIG. 5 shows the heat dissipation calculating section 33 in the clutch temperature estimation calculation process section 31. FIG. 6(a) shows one example of a line pressure sensitivity map, and FIG. 6(b) shows one example of a surface pressure sensitivity map. FIG. 7 shows one example of a heat dissipation quantity map. The heat dissipation calculation construction is explained hereinbelow with reference to FIGS. 5~7.

As shown in FIG. 5, the heat dissipation calculating section 33 includes a lubrication quantity calculating section 33a, a clutch temperature estimating section 33b, a first heat release coefficient calculating section 33c, a product calculating or accumulating section 33d, a heat dissipation quantity calculating section 33e and a dividing section 33f.

The lubrication quantity calculating section 33a calculates a lubrication quantity f(L) around the friction material by multiplication of a line pressure sensitivity f(PL) from a line pressure sensitivity calculating section 33a1 and a surface pressure sensitivity f(β) from a surface pressure sensitivity calculating section 33a2, according to a following equation.

$$f(L) = f(β) \times f(PL)$$

The line pressure sensitivity f(PL) of the lubrication quantity is a sensitivity representing a tendency of the lubrication quantity increasing with increase of the line pressure PL. The line pressure sensitivity f(PL) is determined by sensing (or estimating) the line pressure PL, and using a line pressure sensitivity map (formed on the basis of experiment data) shown in FIG. 6(a). The line pressure sensitivity f(PL) of the lubrication quantity is given in proportion to the level of the line pressure PL. Though the line pressure sensitivity does not vary linearly with a linear characteristic, the line pressure sensitivity increases as the line pressure PL increases.

The surface pressure sensitivity f(β) of the lubrication quantity is a sensitivity representing a tendency of the lubrication quantity decreasing with decrease of gap(s) among the plates. The surface pressure sensitivity f(β) is determined by reading a solenoid regulation pressure f(Pc) and using a surface pressure sensitivity map shown in FIG. 6(b). The surface pressure sensitivity f(β) is given by the degree of separation or opening of the adjacent friction material members. The surface pressure sensitivity f(β) is set equal to a highest value during the disengagement, and equal to a lowest value during the engagement. During the slip, the surface pressure sensitivity f(β) is decreased gradually from the value of the disengagement to the value of the engagement.

The clutch temperature estimating section 33b reads a previous clutch temperature Tpv which is a previous value of the clutch temperature estimated by the previous operaton, and sets this temperature as an estimated clutch temperature.

The clutch temperature estimating section 33b may be configured to calculate a temperature difference ΔT according to a following equation, since the temperature difference is a parameter having such a characteristic that the heat dissipation effect is higher as the temperature difference is higher. The previous clutch temperature Tpv is used since the previous clutch temperature represents the temperature difference when the ATF oil temperature is assumed to be constant.

$$ΔT = Tpv - AFTtemp$$

where, Tpv: previous clutch temperature, and ATFtemp: ATF oil temperature.

The first heat release coefficient calculating section 33c calculates a first heat release coefficient according to a following mathematical expression,

$$C \times α$$

where C: the specific heat, and α: an effective lubrication flow quantity coefficient. The effective lubrication flow quantity coefficient α is determined by a following equation,

$$α = \text{engagement surface pressure/average surface pressure}$$

The "engagement surface pressure or surface pressure at the time of engagement (slip engagement ~ full engagement)" is a surface pressure produced in clutch 23, in dependence on the clutch oil pressure. The "average surface pressure" is an average surface pressure in a clutch operating environment (for example, the surface pressure at an oil pressure equaling a greatest oil pressure/2). Since the engagement surface pressure varies in accordance with the clutch oil pressure, the effective lubrication flow quantity coefficient α is equal to one when the engagement surface pressure is equal to the average surface pressure. The effective lubrication flow quantity coefficient α is equal to a value (<1) advantageous for the heat dissipation when the engagement surface pressure is on a lower pressure side of the average surface pressure. The effective lubrication flow quantity coefficient α is equal to a value (>1) disadvantageous for the heat dissipation when the engagement surface pressure is on a higher pressure side of the average surface pressure.

The product calculating section 33d determines a product of the lubrication quantity f(L) and the previous clutch temperature Tpv. The product serves as an indicator representing the heat dissipating effect since the heat dissipating effect is greater due to heat transfer from the friction material to the lubricating oil as the lubrication quantity f(L) is greater, and the heat dissipating effect is greater due to heat transfer from the friction material to the lubricating oil as the previous clutch temperature Tpv is higher.

The heat dissipation quantity calculating section 33e determines a map selection coefficient B by using information on the current state of clutch 23 among the states of engagement, slipping and disengagement, the previous clutch temperature Tpv and a heat dissipation quantity map shown in FIG. 7. The heat dissipation quantity calculating section 33e calculates the heat dissipation quantity f(t) by a value of product of the map selection coefficient B, the lubricating oil quantity f(L) and the previous clutch temperature Tpv according to a following equation.

$$f(t) = B \times Tpv \times f(L)$$

As shown in FIG. 7, the map selection coefficient B is a quantity to select a value of the heat dissipation coefficient corresponding to the clutch temperature and the clutch state, and the slopes or gradients are determined in dependence on the clutch state so that B1 (during full engagement) < B2 (during slip engagement) < B3 (during disengagement).

The dividing section 33f calculates the temperature decrease ΔT(down) due to the heat dissipation by diving the heat dissipation quantity f(t) by the first heat release coefficient according to a following equation.

$$\Delta T(down) = (f(t) \times \Delta t / 1000) / C \times \alpha \qquad \cdots (2)$$

where Δt is the calculating cycle. The temperature decrease ΔT(down) represents a temperature decrease per unit time determined by the calculating cycle Δt.

From the equations (1) and (2), the temperature variation ΔT per unit time is given by a following equation.

$$\Delta T = \Delta T(up) - \Delta T(down)$$
$$= \{\Delta N \times \mu ND(APc - F) \times \Delta t / 1000\} / C \times K - (f(t) \times \Delta t / 1000) / C \times \alpha$$
$$\cdots (3)$$

[Improvement of Clutch Temperature Estimation Accuracy]

As mentioned above, according to the embodiment 1, the heat dissipation calculating section 33 is configured to estimate the lubricating oil quantity which is a quantity of the lubricating oil passing through the circumstance of the friction material of clutch 23, and to calculate the heat dissipation quantity f(t) which a quantity of heat dissipated from the friction material to the lubricating oil by heat transfer, in accordance with the estimated lubricating oil quantity.

When the lubricating oil flows around the friction material of clutch 23, the frictional heat generated in the friction material is released or dissipated to the lubricating oil by the action of heat transfer from the friction material to the lubricating oil. If the temperature decrease due to this heat dissipation is not taken into consideration, the estimated clutch temperature would be too high.

By taking into the heat dissipation calculation, the lubricating oil quantity of the lubricating oil flowing around the friction material, by contrast, the heat dissipation from the friction material to the lubricating oil by heat transfer is reflected in the estimation of the temperature clutch 23 by the heat generation calculation and the heat dissipation calculation. Thus, it is possible to improve the accuracy of the temperature estimation for clutch 23 by taking into consideration, the effect of the heat dissipation by heat transfer from the friction material to the lubricating oil.

[Improvement of Accuracy in Calculation of Temperature Decrease due to Heat Dissipation]

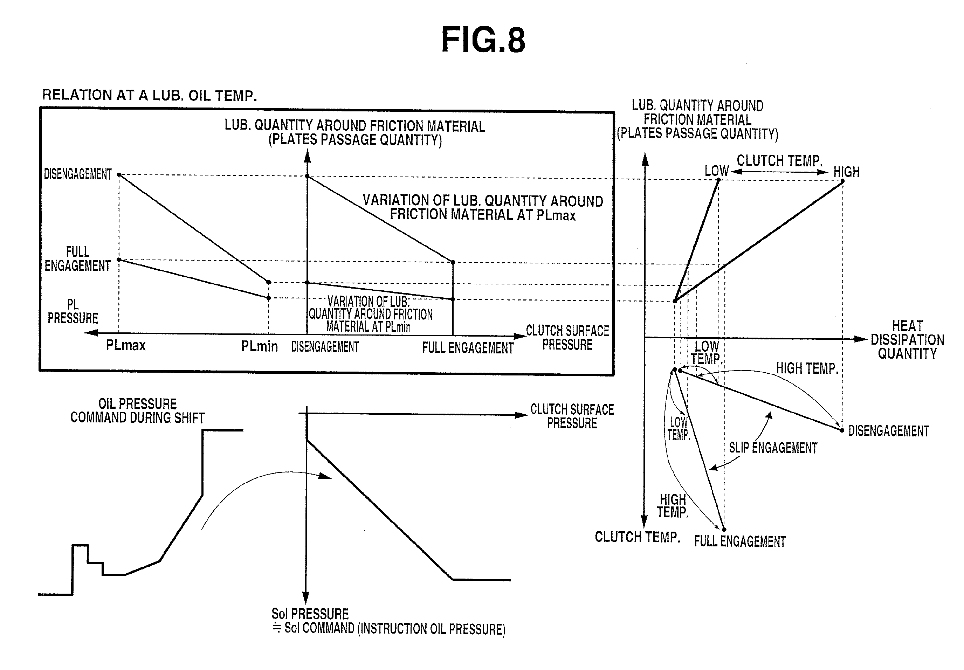

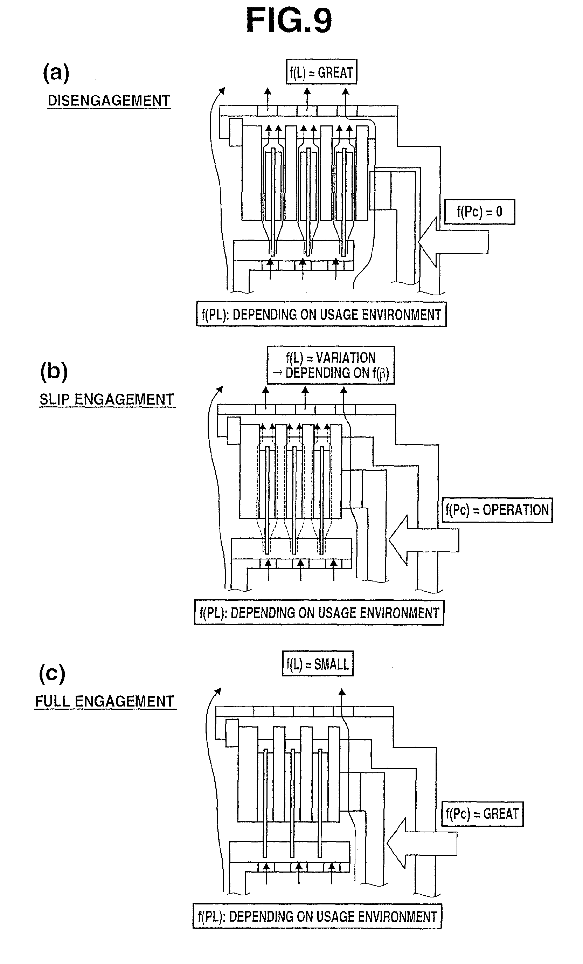

In order to improve the calculation accuracy of temperature decrease ΔT(down) in heat dissipation calculating section 33, it is required to figure out the quantity of the lubricating oil passing around the friction material and effecting the heat dissipation, accurately. FIGS. 8 and 9 illustrate the improvement of the accuracy in the calculation of the temperature decrease due to the heat dissipation.

FIG. 8 shows a relation of factors to estimate the lubricating oil quantity, to the lubrication quantity around the friction material (the quantity of oil passing through the plates, or plates passage quantity), used as the base for the heat dissipation calculation.

A relationship, at a certain lubricating oil temperature, between the lubrication quantity around the friction material and the clutch surface pressure is shown in a frame in FIG. 8. As shown by variation or transition characteristics of the lubrication quantity around the friction material at a maximum line pressure PLmax and a minimum line pressure PLmin in the frame, the lubrication quantity is greatest at the clutch surface pressure in the disengagement state, and the lubrication quantity is smallest at the clutch surface pressure in the full engagement state. At the surface pressure state between the disengagement state and the full engagement state, the lubrication quantity becomes gradually smaller from the disengagement state toward the full engagement state. As shown by a shift time oil pressure command characteristic and a clutch surface pressure characteristic in FIG. 8, the clutch surface pressure can be determined from a solenoid command at the time of shift. On the other hand, as shown by the characteristic of the line pressure in the disengagement state and the characteristic of the line pressure in the full engagement state in the frame of FIG. 8, the relationship between the lubrication quantity around the friction material and the line pressure (PL pressure) at the certain lubricating oil temperature is such that the lubrication quantity is greatest at the maximum line pressure PLmax and the lubrication quantity is smallest at the minimum line pressure PLmin. In the line pressure state therebetween, the lubrication quantity becomes gradually smaller from the maximum line pressure PLmax toward the minimum line pressure PLmin. Therefore, in the estimation of the lubrication quantity around the friction material, the lubricating oil quantity estimating factors are: "clutch surface pressure", "line pressure PL" and "lubricating oil temperature (lubricating oil viscosity)".

A relationship between the lubrication quantity around the friction material (the quantity of oil passing through the plates) and the heat dissipation quantity when the clutch temperature is varied, is shown by characteristics on the right side of the frame in FIG. 8. As shown by these characteristics, the heat dissipation quantity becomes greater as the lubrication quantity around the friction material becomes greater. When the clutch temperature is varied from a lower temperature to a higher temperature, the heat dissipation quantity becomes greater as the clutch temperature becomes higher.

Therefore, in the estimation of the heat dissipation quantity around the friction material, "clutch temperature" is a factor for the estimation. Instead of "clutch temperature", it is possible to use "the temperature difference between the clutch temperature and the ATF oil temperature". In this case, the heat dissipation quantity becomes greater as the temperature difference increases.

In this way, as shown by a relational characteristic shown in a right lower portion in FIG. 8, the heat dissipation quantity is related to the clutch temperature. Specifically, in the clutch disengagement state, the heat dissipation quantity increases with a greater slope or gradient with increase in the clutch temperature. In the clutch full engagement state, the heat dissipation quantity increases with a smaller slope or gradient with increase in the clutch temperature. During the slip engagement, the heat dissipation quantity increases with an intermediate slope or gradient between the slope of the full engagement and the slope of the disengagement, with increase in the clutch temperature.

As explained above, the embodiment 1 employs the structure to estimate the lubricating oil quantity of the lubricating oil passing through the circumference of the friction material in the heat dissipation calculation so that the estimated lubricating oil quantity is greater as the line pressure serving as the original pressure for the engagement pressure supplied to clutch 23 becomes higher.

Accordingly, the relationship of the lubrication quantity increasing with increase in the line pressure PL is reflected in the heat dissipation calculation, and the temperature decrease ΔT(down) due to the heat dissipation can be estimated accurately in accordance with the line pressure PL.

The embodiment 1 employs the structure to estimate the lubricating oil quantity of the lubricating oil passing through the circumstance of the friction material so that the estimated lubricating oil quantity becomes greater as the gap(s) among the friction material is wider. In the clutch disengagement state, as shown in FIG. 9(a), the gaps or clearances among the members of the friction material are wider. Accordingly, the lubrication quantity f(L) becomes greatest. In the slip engagement state, as shown in FIG. 9(b), the gap(s) among the members of the friction material are varied, and the lubrication quantity f(L) becomes smaller as the gap(s) of the friction material become narrower. In the full engagement state, as shown in FIG. 9(c), the gap(s) of the friction material are null, and hence the lubrication quantity f(L) becomes smallest.

Therefore, the relationship of the lubrication quantity f(L) increasing with increase in the gap(s) of the friction material is reflected in the heat dissipation calculation, and the temperature decrease ΔT(down) due to the heat dissipation can be estimated accurately in accordance with the friction material gap.

The embodiment 1 employs the structure to estimate the heat dissipation quantity by heat transfer from the friction material to the lubricating oil in the heat dissipation calculation so that the heat dissipation quantity is greater as the estimated temperature of clutch 23 (the previous clutch temperature Tpv) is higher.

Therefore, the relationship of the heat dissipation quantity increasing with increase in the clutch temperature is reflected in the heat dissipation calculation, and the temperature decrease $\Delta T$(down) due to the heat dissipation can be estimated accurately in accordance with the clutch temperature.

The embodiment 1 employs the structure which includes the first heat release coefficient calculating section 33c for calculating the first heat release coefficient ($C \times \alpha$) representing the quantity of heat dissipation by heat transfer from the friction material of clutch 23 to the lubricating oil, and which is configured to perform a corrective calculation to calculate or correct the heat dissipation quantity f(t) in accordance with the first heat release coefficient ($C \times \alpha$).

Therefore, the relationship of the heat dissipation quantity increasing with increase in the lubrication quantity around the friction material because of a lower clutch surface pressure is reflected in the heat dissipation calculation, and the temperature decrease $\Delta T$(down) due to the heat dissipation can be estimated accurately in accordance with the clutch surface pressure.

The embodiment 1 employs the structure to calculate the first heat release coefficient ($C \times \alpha$) by multiplying the specific heat C determined by the material of the friction material by the effective lubrication flow quantity coefficient $\alpha$ calculated by dividing the surface pressure at the time of engagement by the average surface pressure.

The effective lubrication flow quantity coefficient $\alpha$ is set equal to a value (<1) advantageous for the heat dissipation on the lower surface pressure side lower than the average surface pressure, and set equal to a value (>1) disadvantageous for the heat dissipation on the higher surface pressure side higher than the average surface pressure. In the lubrication quantity of the lubricating oil flowing around the friction material, the effective lubrication flow quantity is a quantity of the lubricating oil used for the heat dissipation. The effective lubrication flow quantity used for the heat dissipation is estimated higher in the lower clutch surface pressure region, and estimated lower in the higher clutch surface pressure region, so that the estimation can be matched more accurately to the measurement.

Therefore, the temperature decrease $\Delta T$(down) due to the heat dissipation can be estimated accurately by taking account of the effective lubrication quantity which the quantity of the lubricating oil used for the heat dissipation in the lubrication quantity around the friction material.

[Improvement of Accuracy in Calculation of Temperature Increase by Heat Generation]

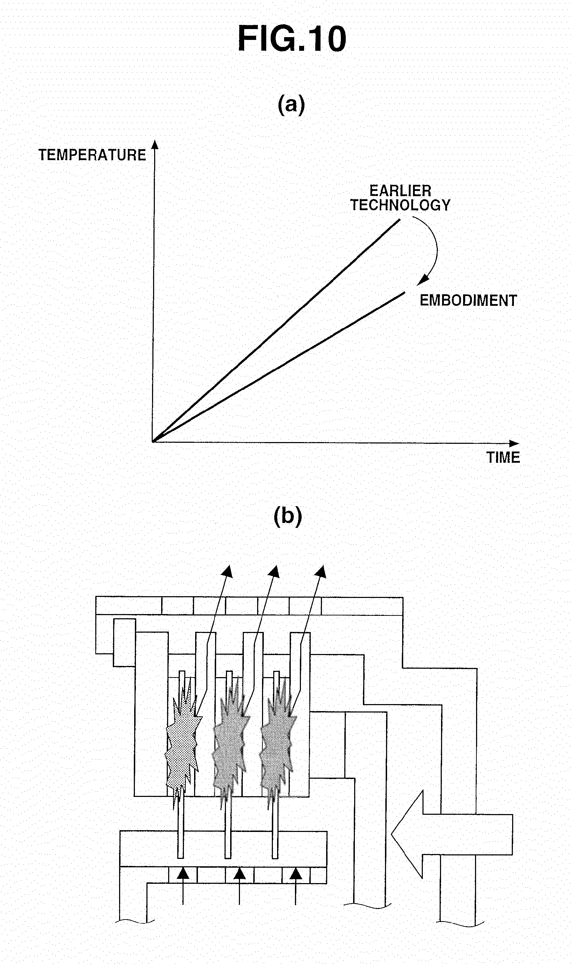

In order to improve the calculation accuracy of temperature increase $\Delta T$(up) in heat generation calculating section 32, it is required to take account of the quantity of heat dissipated or released to the outside by the heat transfer from the friction material which is generating heat, to the base plate(s). FIG. 10 illustrates the improvement of the accuracy in the calculation of the temperature decrease due to the heat release during the heat generation.

The embodiment 1 uses the second heat release coefficient calculating section 32c to calculate the second heat release coefficient (CxK) representing the quantity of heat released or dissipated to the outside by the heat transfer to the base plate(s) during the heat generation of the friction material, and employs the structure to perform a calculation for decrease correction of the heat generation quantity $\Sigma$t in the heat generation calculation with increase in the second heat release coefficient (CxK).

By calculating the temperature increase $\Delta T$(up) by diving the heat generation quantity $\Sigma$t by the second heat release coefficient (CxK), as shown in FIG. 10(a), the increasing gradient of the clutch temperature characteristic with respect to time is made smaller in the embodiment 1 as compared to the increasing gradient in the case of an earlier technology, and hence the temperature increase can be estimated more accurately. The sliding surface heat transfer coefficient K in the second heat release coefficient (CxK) is a coefficient representing the quantity of heat released from clutch 23 to the outside by the heat transfer through the base plate(s) during the heat generation by slipping friction, as shown in FIG. 10(b).

Therefore, the temperature increase $\Delta T$(up) due to the heat generation can be estimated accurately in the heat generation calculation by taking account of the quantity of heat released to the outside by the heat transfer to the base plate(s) during the heat generation of the friction material of clutch 23.

Following is explanation on effects. The calculating apparatus of estimating the temperature of the friction engagement element according to the embodiment 1 can provide following effects.

(1) The friction engagement element (clutch 23) is a device disposed in a driving force transmitting system of a vehicle, and arranged to have operating states of full engagement, slip or slipping engagement and disengagement, and there are provided:

a heat generation calculating means (heat generation calculating section 32) configured to calculate a per-unit-time temperature increase ΔT(up) in accordance with a heat generation quantity Σt which a quantity of heat generated among friction material members of the friction engagement element (clutch 23);

a heat dissipation calculating means (heat dissipation calculating section 33) configured to calculate a per-unit-time temperature decrease ΔT(down) in accordance with a heat dissipation quantity f(t) which is a quantity of heat dissipated from the friction material member(s) of the friction engagement element (clutch 23); and

a friction engagement element estimated temperature calculating means (clutch temperature estimation calculating section 31) configured to set a temperature difference resulting from subtraction of the temperature decrease ΔT(down) from the temperature increase ΔT(up), as a per-unit-time temperature variation ΔT, and to calculate a current friction engagement element estimated temperature (current clutch temperature Tnow) by adding the temperature variation ΔT to a previous estimated temperature (previous clutch temperature Tpv) of the friction engagement element (clutch 23) obtained by a previous calculating cycle.

The heat dissipation calculating means (heat dissipation calculating section 33) is configured to estimate a lubricating oil quantity of a lubricating oil passing through the circumference or surrounding space of the friction material member(s) of the friction engagement element (clutch 23), and to calculate the heat dissipation quantity f(t) of heat dissipated or released from the friction material member(s) to the lubricating oil by heat transfer, in accordance with the estimated lubricating oil quantity.

Therefore, the calculating apparatus for estimating the temperature of the friction engagement element can improve the accuracy in estimating the temperature of the friction engagement element (clutch 23) by taking account of the heat dissipating effect by the lubricating oil flowing around the friction material.

(2) The heat dissipation calculating means (heat dissipation calculating section 33) is configured to estimate the lubricating oil quantity of the lubricating oil passing around the friction material in such a manner that the estimated lubricating oil quantity is increased as the line pressure PL serving as an original pressure for an engagement pressure supplied to the friction engagement element is increased.

Therefore, in addition to the effect (1), the calculating apparatus can take account of the relationship of the lubrication quantity increasing as the line pressure becomes higher, in the heat dissipation calculation, and estimate the temperature decrease Δt(down) caused by the heat dissipation accurately in accordance with the line pressure PL.

(3) The heat dissipation calculating means (heat dissipation calculating section 33) estimates the lubricating oil quantity f(L) of the lubricating oil passing around the friction material in such a manner that the estimated lubricating oil quantity f(L) becomes greater as the gap(s) or clearance(s) among the adjacent portions of the friction material is wider. Therefore, in addition to the effects (1) and (2), the calculating apparatus can perform the heat dissipation calculation so that the relationship of the lubricating oil quantity f(L) increasing with increase in the gap(s) of the friction material is reflected in the heat dissipation calculation, and estimate the temperature decrease Δt(down) caused by the heat dissipation accurately in accordance with the gap(s) of the friction material.

(4) The heat dissipation calculating means (heat dissipation calculating section 33) is configured to estimate the heat dissipation quantity of heat dissipated from the friction material to the lubricating oil by heat transfer so that the estimated heat dissipation quantity is greater as the estimated temperature (previous clutch temperature Tpv) of the friction engagement element (clutch 23) is higher.

Therefore, in addition to the effects (1)~(3), the calculating apparatus can perform the heat dissipation calculation so that the relationship of the heat dissipation quantity increasing with increase in the clutch temperature is reflected in the heat dissipation calculation, and estimate the temperature decrease Δt(down) caused by the heat dissipation accurately in accordance with the clutch temperature.

(5) A first heat release coefficient calculating means (first heat release coefficient calculating section 33c) is provided and configured to calculate a first heat release coefficient (C×α) representing a quantity of heat dissipated or released by heat transfer from the friction material of the friction engagement element (clutch 23) to the lubricating oil, and the heat dissipation calculating means (heat dissipation calculating section 33) is configured to correct the heat dissipation quantity in accordance with the magnitude of the first heat release coefficient (C×α).

Therefore, in addition to the effects (1)~(4), the calculating apparatus can perform the heat dissipation calculation so that the relationship of the heat dissipation quantity being increased as the clutch surface pressure is lower and the lubrication quantity around the friction material is greater, is reflected in the heat dissipation calculation, and estimate the temperature decrease Δt(down) caused by the heat dissipation accurately in accordance with the magnitude of the clutch surface pressure.

(6) The first heat release coefficient calculating means (first heat release coefficient calculating section 33) is configured to calculate the first heat release coefficient (C×α) by a product of a specific heat C determined by the material of the friction material and an effective lubricating flow quantity coefficient α calculated by dividing an engagement time surface pressure or the surface pressure at the time of engagement by an average surface pressure.

Therefore, in addition to the effect (5), the calculating apparatus can take account of the effective lubrication flow quantity used for the heat dissipation in the lubrication quantity flowing around the friction material, and thereby estimate the temperature decrease ∆T(down) due to the heat dissipation accurately.

(7) A second heat release coefficient calculating means (second heat release coefficient calculating section 32c) is provided and configured to calculate a second heat release coefficient (CxK) representing a quantity of heat released to the outside by heat transfer to base plate(s) during the heat generation of the friction material of the friction engagement element (clutch 23), and the heat generation calculating means (heat generation calculating section 32) is configured to decrease the heat generation quantity Σt as the second heat release coefficient becomes higher. Therefore, in addition to the effects (1)~(6), the calculating apparatus can perform the heat generation calculation by taking account of the quantity of heat released to the outside by the heat transfer to the base plate(s) during the heat generation of the friction material of the friction engagement element (clutch 23), and thereby estimate the temperature increase ∆T(up) due to the heat generation accurately.

Although the friction engagement element temperature estimation calculating apparatus according to the present invention has been described above with reference to the embodiment 1, the concrete construction is not limited to the embodiment 1, and change of design, addition and other variation are permissible within the purview of the invention defined by the subject matter of the claims.

As the friction engagement element whose temperature is to be estimated, the first embodiment 1 employs, as an example, the clutch(s) 23 included as a shifting device in the automatic transmission 1. However, the friction engagement element used for the temperature estimation may be a lockup clutch used for slip lockup control, or a clutch for a hybrid vehicle for performing a slip control at the time of a start of vehicle, a start of the engine and/or EV driving operation. The present invention is applicable to any friction engagement element which is disposed in a driving force transmitting path of a vehicle, and which is arranged to be operated in operating states of full engagement, slipping or slip engagement and disengagement.

As the heat generation calculating means, the embodiment 1 employs, as an example, the heat generation calculating section 32 to calculate the per-unit-time temperature increase ∆T(up) in accordance with the heat generation quantity Σt of heat generated between the friction material members of clutch 23 and the second heat release coefficient (CxK). However, the heat generation calculating means may be a means to calculate the per-unit-time temperature increase in accordance with the heat generation quantity of heat generated between the friction material members of the clutch.

As the heat dissipation calculating means, the embodiment 1 employs, as an example, the heat dissipation calculating section 33 to calculate the per-unit-time temperature decrease ∆T(down) in accordance with the heat dissipation quantity f(t) of heat dissipated from the friction material of clutch 23 and the first heat release coefficient (C×α). However, the concrete construction of the heat dissipation calculation is not limited to the heat dissipation calculating section 33 of the embodiment 1 as long as the heat dissipation calculating means is configured to estimate the lubricating oil quantity of the lubricating oil passing around the friction material of the clutch and to calculate the heat dissipation quantity f(t) of heat dissipated by heat transfer from the friction material to the lubricating oil. For example, the method or system of estimating the quantity of lubricating oil passing around the friction material of the clutch may be arranged to use part of the lubrication estimating factors shown in the embodiment 1, or use factor(s) other than the lubrication estimating factors shown in the embodiment 1.

## Claims

1. A temperature estimation calculating apparatus to calculate an estimated temperature of a friction engagement element which is disposed in a driving force transmitting system of a vehicle, and which is arranged to attain operating states of full engagement, slip engagement and disengagement, the temperature estimation calculating apparatus comprising:

a heat generation calculating means to calculate a temperature increase per unit time in accordance with a heat generation quantity of heat generated between portions of friction material of the friction engagement element; a heat dissipation calculating means to calculate a temperature decrease per unit time in accordance with a heat dissipation quantity of heat dissipated from the friction material of the friction engagement element; and a friction engagement element estimated temperature calculating means to set a temperature difference resulting from subtraction of the temperature decrease from the temperature increase, as a temperature variation per unit time, and to calculate a current friction engagement element estimated temperature by adding the temperature variation to a previous friction engagement estimated temperature estimated until a previous calculating cycle;

the heat dissipation calculating means being configured to estimate a lubricating oil quantity of a lubricating oil passing around the friction material of the friction engagement element, and to calculate the heat dissipation quantity of heat dissipated from the friction material to the lubricating oil by heat transfer, in accordance with the lubricating oil quantity obtained by estimation.

2. The temperature estimation calculating apparatus as claimed in Claim 1, wherein the heat dissipation calculating means is configured to estimate the lubricating oil quantity of the lubricating oil passing around the friction material so that the lubricating oil quantity becomes greater as a line pressure as an original pressure for an engagement pressure supplied to the friction engagement element is higher.

3. The temperature estimation calculating apparatus as claimed in Claim 1 or 2, wherein the heat dissipation calculating means is configured to estimate the lubricating oil quantity of the lubricating oil passing around the friction material so that the lubricating oil quantity becomes greater as a gap between adjacent portions of the friction material is wider.

4. The temperature estimation calculating apparatus as claimed in one of Claims 1~3, wherein the heat dissipation calculating means is configured to estimate the heat dissipation quantity of heat dissipated from the friction material to the lubricating oil by heat transfer so that the heat dissipation quantity is greater as the estimated temperature of the friction engagement element is higher.

5. The temperature estimation calculating apparatus as claimed in one of Claims 1~4, wherein a first heat release coefficient calculating means is provided and configured to calculate a first heat release coefficient representing a quantity of heat released by heat transfer from the friction material of the friction engagement element to the lubricating oil, and the heat dissipation calculating means is configured to correct the heat dissipation quantity in accordance with the first heat release coefficient.

6. The temperature estimation calculating apparatus as claimed in Claim 5, wherein the first heat release coefficient calculating means is configured to calculate the first heat release coefficient by a product of a specific heat determined by the friction material and an effective lubricating flow quantity coefficient calculated by dividing an engagement time surface pressure by an average surface pressure.

7. The temperature estimation calculating apparatus as claimed in one of Claims 1~6, wherein a second heat release coefficient calculating means is provided and configured to calculate a second heat release coefficient representing a quantity of heat released to an outside by heat transfer to a base plate(of the clutch engagement element) during the heat generation of the friction material of the friction engagement element, and the heat generation calculating means is configured to decrease the heat generation quantity as the second heat release coefficient increases.


**Amended claims under Art. 19.1 PCT**

1. (Amended) A temperature estimation calculating apparatus to calculate an estimated temperature of a friction engagement element which is disposed in a driving force transmitting system of a vehicle, and which is arranged to attain operating states of full engagement, slip engagement and disengagement, the temperature estimation calculating apparatus comprising:

a heat generation calculating means to calculate a temperature increase per unit time in accordance with a heat generation quantity of heat generated between portions of friction material of the friction engagement element; a heat dissipation calculating means to calculate a temperature decrease per unit time in accordance with a heat dissipation quantity of heat dissipated from the friction material of the friction engagement element; and a friction engagement element estimated temperature calculating means to set a temperature difference resulting from subtraction of the temperature decrease from the temperature increase, as a temperature variation per unit time, and to calculate a current friction engagement element estimated temperature by adding the temperature variation to a previous friction engagement estimated temperature estimated until a previous calculating cycle;
the heat dissipation calculating means being configured to estimate a lubricating oil quantity of a lubricating oil passing around the friction material of the friction engagement element, and to calculate the heat dissipation quantity of heat dissipated from the friction material to the lubricating oil by heat transfer, in accordance with the lubricating oil quantity obtained by estimation;
wherein a first heat release coefficient calculating means is provided and configured to calculate a first heat release coefficient representing a quantity of heat released by heat transfer from the friction material of the friction engagement element to the lubricating oil, and
the heat dissipation calculating means is configured to correct the heat dissipation quantity in accordance with the first heat release coefficient.

**2.** The temperature estimation calculating apparatus as claimed in Claim 1, wherein the heat dissipation calculating means is configured to estimate the lubricating oil quantity of the lubricating oil passing around the friction material so that the lubricating oil quantity becomes greater as a line pressure as an original pressure for an engagement pressure supplied to the friction engagement element is higher.

**3.** The temperature estimation calculating apparatus as claimed in Claim 1 or 2, wherein the heat dissipation calculating means is configured to estimate the lubricating oil quantity of the lubricating oil passing around the friction material so that the lubricating oil quantity becomes greater as a gap between adjacent portions of the friction material is wider.

**4.** The temperature estimation calculating apparatus as claimed in one of Claims 1~3, wherein the heat dissipation calculating means is configured to estimate the heat dissipation quantity of heat dissipated from the friction material to the lubricating oil by heat transfer so that the heat dissipation quantity is greater as the estimated temperature of the friction engagement element is higher.

**5.** (Amended) The temperature estimation calculating apparatus as claimed in one of Claims 1-4, wherein the first heat release coefficient calculating means is configured to calculate the first heat release coefficient by a product of a specific heat determined by the friction material and an effective lubrication flow quantity coefficient calculated by dividing an engagement time surface pressure by an average surface pressure.

**6.** (Amended) A temperature estimation calculating apparatus to calculate an estimated temperature of a friction engagement element which is disposed in a driving force transmitting system of a vehicle, and which is arranged to attain operating states of full engagement, slip engagement and disengagement, the temperature estimation calculating apparatus comprising:

a heat generation calculating means to calculate a temperature increase per unit time in accordance with a heat generation quantity of heat generated between portions of friction material of the friction engagement element;
a heat dissipation calculating means to calculate a temperature decrease per unit time in accordance with a heat dissipation quantity of heat dissipated from the friction material of the friction engagement element; and
a friction engagement element estimated temperature calculating means to set a temperature difference resulting from subtraction of the temperature decrease from the temperature increase, as a temperature variation per unit time, and to calculate a current friction engagement element estimated temperature by adding the temperature variation to a previous friction engagement estimated temperature estimated until a previous calculating cycle;
the heat dissipation calculating means being configured to estimate a lubricating oil quantity of a lubricating oil passing around the friction material of the friction engagement element, and to calculate the heat dissipation quantity of heat dissipated from the friction material to the lubricating oil by heat transfer, in accordance with the lubricating oil quantity obtained by estimation ;
wherein a second heat release coefficient calculating means is provided and configured to calculate a second heat release coefficient representing a quantity of heat released to an outside by heat transfer to a base plate during heat generation of the friction material of the friction engagement element, and the heat generation calculating means is configured to decrease the heat generation quantity as the second heat release coefficient increases.

**7.** (Amended) The temperature estimation calculating apparatus as claimed in Claim 6, wherein the heat dissipation calculating means is configured to estimate the lubricating oil quantity of the lubricating oil passing around the friction material so that the lubricating oil quantity becomes greater as a line pressure used as an original pressure for an engagement pressure supplied to the friction engagement element is higher.

**8.** Added) The temperature estimation calculating apparatus as claimed in Claim 6 or 7, wherein the heat dissipation calculating means is configured to estimate the lubricating oil quantity of the lubricating oil passing around the friction material so that the lubricating oil quantity becomes greater as a gap between adjacent portions of the friction material is wider.

**9.** Added) The temperature estimation calculating apparatus as claimed in one of Claims 6~8, wherein the heat dissipation calculating means is configured to estimate the heat dissipation quantity of heat dissipated from the friction material to the lubricating oil by heat transfer so that the heat dissipation quantity is greater as the estimated temperature of the friction engagement element is higher.

**10.** Added) The temperature estimation calculating apparatus as claimed in one of Claims 6~9, wherein the first heat release coefficient calculating means is configured to calculate the first heat release coefficient by a product of a specific heat determined by the friction material and an effective lubricating oil quantity coefficient calculated by dividing an engagement time surface pressure by an average surface pressure.

# FIG.1

AT

Eng

AT CONTROLLER

SHIFT CONTROL PROCESS SECTION

HEAT GENERATION CALCULATING SECTION

HEAT DISSIPATION CALCULATING SECTION

IGN-SW

VEHICLE SPEED SENSOR

ACCELERATOR OPENING SENSOR

TURBINE ROTATION SPEED SENSOR

OUTPUT ROTATION SPEED SENSOR

LINE PRESSURE SENSOR

ATF OIL TEMPERATURE SENSOR

# FIG.2

START

$Tpv = $ ATF OIL TEMPERATURE — S0

$Tnow = Tpv + \Delta T(up) - \Delta T(down)$ — S1

CLUTCH STATE OF FULL ENGAGEMENT OR DISENGAGEMENT? — S2
No → CLUTCH STATE OF SLIP ENGAGEMENT? — S4
No → CLUTCH SHIFT PROCESS? — S7
No

Yes

Yes → HEAT GENERATION CALCULATION ($\Delta T(up)$) — S5

Yes → HEAT GENERATION CALCULATION ($\Delta T(up)$) — S8

S3 — HEAT DISSIPATION CALCULATION ($\Delta T(down)$)

S6 — HEAT DISSIPATION CALCULATION ($\Delta T(down)$)

HEAT DISSIPATION CALCULATION ($\Delta T(down)$) — S9

S10 — IGN-OFF?
No

Yes

END

EP 2 949 957 A1

# FIG.3

| | | | |
|---|---|---|---|
| μ | | | |
| N | | | |
| D | → | **CLUTCH TORQUE CALCULATION** *32a* | |
| A | | $Tc = \mu \times N \times D \times (APc - F)$ | |
| Pc | | | |
| F | | | |

**32d** × → **32e** ÷ → ΔT(up): TEMPERATURE INCREASE

**CLUTCH RELATIVE ROTATION SPEED CALCULATION** *32b*

No →
Nt →

$\Delta N = a \times No + b \times Nt$

(a, b depend on gear speed, friction engagement element)

**SECOND HEAT RELEASE COEFFICIENT CALCULATION** *32c*

C →
K →

$C \times K$

μ: FRICTION COEFFICIENT OF FRICTION MATERIAL
N: NUMBER OF CLUTCH PLATES
D: EFFECTIVE DIAMETER OF FRICTION MATERIAL
A: PISTON PRESSURE RECEIVING AREA
Pc: CLUTCH PRESSURE
F: RETURN SPRING LOAD
Nt: TURBINE ROTATION SPEED
No: OUTPUT ROTATION SPEED
C: SPECIFIC HEAT
K: SLIDING SURFACE HEAT TRANSFER COEFFICIENT

# FIG.4

t1    t2    t3 t4    PL

f(Pc)

ACCUMULATION OF HEAT

# FIG.5

CALCULATION OF LUBRICATION
QUANTITY AROUND FRICTION MATERIAL
33a

$f(L) = f(\beta) \times f(PL)$

33a1

LINE PRESSURE (PL)
→ LINE PRESSURE SENSITIVITY f(PL)
CALCULATION

33a2

SHIFT COMMAND f(Pc)
→ SURFACE PRESSURE SENSITIVITY f(β)
CALCULATION

33

33d
×

CALCULATION OF HEAT
DISSIPATION QUANTITY

$f(t) = B \times Tpv \times f(L)$

B: map selection coefficient
(to select heat dissipation gradient
corresponding to clutch temperature)

33e

33f
÷

ΔT(down): TEMPERATURE DECREASE

PREVIOUS CLUTCH PRESSURE (Tpv)

33b

C

α

FIRST HEAT RELEASE
COEFFICIENT CALCULATION
33c

$C \times \alpha$

PL: LINE PRESSURE
Pc: CLUTCH PRESSURE
Tpv: PREVIOUS CLUTCH
TEMPERATURE
C: SPECIFIC HEAT
α: EFFECTIVE LUBRICATION
FLOW COEFFICIENT
β: CLUTCH SURFACE PRESSURE
B: MAP SELECTION COEFFICIENT

EP 2 949 957 A1

# FIG.6

## (a)

## (b)

# FIG.7

# FIG.8

RELATION AT A LUB. OIL TEMP.

LUB. QUANTITY AROUND FRICTION MATERIAL
(PLATES PASSAGE QUANTITY)

DISENGAGEMENT

VARIATION OF LUB. QUANTITY AROUND
FRICTION MATERIAL AT PLmax

FULL
ENGAGEMENT

PL
PRESSURE

VARIATION OF LUB.
QUANTITY AROUND FRICTION
MATERIAL AT PLmin

CLUTCH SURFACE
PRESSURE

PLmax          PLmin    DISENGAGEMENT        FULL ENGAGEMENT

OIL PRESSURE
COMMAND DURING SHIFT

CLUTCH SURFACE
PRESSURE

Sol PRESSURE
≒ Sol COMMAND (INSTRUCTION OIL PRESSURE)

LUB. QUANTITY AROUND
FRICTION MATERIAL
(PLATES PASSAGE QUANTITY)

CLUTCH TEMP.
LOW          HIGH

HEAT
DISSIPATION
QUANTITY

LOW
TEMP.

HIGH TEMP.

LOW
TEMP.

DISENGAGEMENT

SLIP ENGAGEMENT

HIGH
TEMP.

CLUTCH TEMP.   FULL ENGAGEMENT

EP 2 949 957 A1

# FIG.9

**(a)**
__DISENGAGEMENT__

f(L) = GREAT

f(Pc) = 0

f(PL): DEPENDING ON USAGE ENVIRONMENT

**(b)**
__SLIP ENGAGEMENT__

f(L) = VARIATION
→ DEPENDING ON f(β)

f(Pc) = OPERATION

f(PL): DEPENDING ON USAGE ENVIRONMENT

**(c)**
__FULL ENGAGEMENT__

f(L) = SMALL

f(Pc) = GREAT

f(PL): DEPENDING ON USAGE ENVIRONMENT

# FIG.10

(a)

(b)

## INTERNATIONAL SEARCH REPORT

International application No.

PCT/JP2013/082000

### A. CLASSIFICATION OF SUBJECT MATTER
*F16D25/12*(2006.01)i, *F16H59/72*(2006.01)i

According to International Patent Classification (IPC) or to both national classification and IPC

### B. FIELDS SEARCHED

Minimum documentation searched (classification system followed by classification symbols)
F16D25/12, F16H59/72, F16H61/14, F16H61/02

Documentation searched other than minimum documentation to the extent that such documents are included in the fields searched
Jitsuyo Shinan Koho          1922-1996   Jitsuyo Shinan Toroku Koho   1996-2014
Kokai Jitsuyo Shinan Koho    1971-2014   Toroku Jitsuyo Shinan Koho   1994-2014

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2009-243638 A  (Aisin AW Co., Ltd.),<br>22 October 2009 (22.10.2009),<br>paragraphs [0062] to [0064]<br>(Family: none) | 1-2<br>3-4<br>5-7 |
| Y | JP 2009-79717 A  (JATCO Ltd.),<br>16 April 2009 (16.04.2009),<br>paragraphs [0038] to [0039]<br>& US 2009/0082933 A1     & DE 102008048439 A1 | 3-4 |
| A | JP 2011-149516 A  (Honda Motor Co., Ltd.),<br>04 August 2011 (04.08.2011),<br>paragraphs [0065], [0072]<br>(Family: none) | 1-7 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 February, 2014 (12.02.14) | 25 February, 2014 (25.02.14) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2013/082000

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2005-351349 A (Nissan Motor Co., Ltd.), 22 December 2005 (22.12.2005), paragraphs [0003] to [0006] (Family: none) | 1-7 |
| A | JP 2008-128385 A (Exedy Corp.), 05 June 2008 (05.06.2008), paragraphs [0082] to [0084]; fig. 8 & US 2010/0320050 A1 & WO 2008/062652 A1 | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011099513 A